# EUROPEAN PATENT SPECIFICATION

(11) **EP 2 745 856 B1**
(45) Date of publication and mention of the grant of the patent: **08.03.2017**
(21) Application number: 13197265.5
(22) Date of filing: 13.12.2013
(51) Int. Cl.: A61L 29/16, A61L 31/16

(54) **Bioactive Compositions, Bioactive Eluting Devices and Methods of Use Thereof**
Bioaktive Zusammensetzungen, bioaktive Elutionsvorrichtungen und Verfahren zu ihrer Verwendung
Compositions bioactives, dispositifs d'élution bioactifs et leurs procédés d'utilisation

(30) Priority: 19.12.2012 US 201261739285 P
(43) Date of publication of application: 25.06.2014
(73) Proprietor: Cook Medical Technologies LLC, Bloomington, IN 47404 (US)
(72) Inventor: Pendleton, Steven L., Spencer, Indiana 47460 (US); Williams, Jeffrey D., Spencer, Indiana 47460 (US)
(74) Representative: Garratt, Peter Douglas

(56) References cited:
- US-A1- 2010 324 645
- US-A1- 2011 287 169

## Description

This patent application claims the benefit of United States provisional patent application number 61/739,285, filed December 19, 2012.

### TECHNICAL FIELD

The embodiments relate generally to compositions including a bioactive, bioactive-eluting medical devices and to methods of use thereof. In certain embodiments, the devices are implantable either partly or completely into a human or veterinary patient. The compositions or devices include agents that enhance the delivery of the bioactive to the patient tissue.

### BACKGROUND

It has become common to treat a variety of medical conditions by delivering a bioactive through the wall of a patient vessel. For example, the bioactive may be delivered systemically or locally as a pharmaceutical preparation in a liquid, gel or solid form. Alternatively, an implantable medical device including the bioactive may be partly or completely introduced into the esophagus, trachea, colon, biliary tract, urinary tract, vascular system or other location within a human or veterinary patient. For example, many treatments of the vascular or urinary systems entail the introduction of a device such as a stent, catheter, balloon, wire guide, cannula, or the like.

An implantable vascular stent may include a bioactive for delivery to treat a condition of the patient or to prevent an injury associated with the implantation of the device. For example, when such a device is introduced into and manipulated through the vascular system, the blood vessel walls can be disturbed or injured. Clot formation or thrombosis often results at the injured site, causing stenosis or occlusion of the blood vessel. Moreover, if the medical device is left within the patient for an extended period of time, a thrombus often forms on the device itself, again causing stenosis or occlusion. As a result, the patient is placed at risk of a variety of complications, including heart attack, pulmonary embolism and stroke. Thus, the use of such a medical device can entail the risk of precisely the problems that its use was intended to ameliorate.

Vascular stents may be coated with an elutable bioactive material such as paclitaxel, sirolimus or a sirolimus derivative as a means of overcoming problems associated with damage to the wall of the vascular system caused by the introduction and presence of the stent. Such devices deliver the bioactive material directly into the vessel wall , so as to treat or prevent abrupt closure and/or restenosis of the blood vessel.

Another problem associated with implantable medical devices and, more particularly, to partly implanted medical devices such as catheters percutaneously introduced into the vascular system of a patient for long-term hemodialysis or bioactive infusion is the risk of infection. This risk is also present with hyperalimentation (intravenous feeding) catheters. In addition, similar risks exist in the urinary tract of the patient when an urethral catheter, such as a Foley catheter, is introduced into the patient's bladder via the urethra for the drainage of urine. In an attempt to reduce the risk of such an infection a bioactive, such as an antibiotic, way be used in conjunction with the catheter.

However, in some applications, the lining of the vessel wall may present a robust barrier that prevents or reduces delivery of the bioactive from these compositions or devices to the tissue of the patient. For example, the wall of the urinary tract can present a significant barrier to the delivery of bioactive from devices such as ureteral stents and catheters. Such a problem may be compounded in situations where a device is implanted in a vessel having a high fluid flow, resulting in a portion of the bioactive being carried away from the region under treatment.

### SUMMARY

In one aspect, the present invention provides a composition including triacetin and dimethyl sulfoxide and a bioactive. In certain embodiments, the composition also includes a pharmaceutically acceptable carrier. Another aspect provides an implantable medical device including triacetin, dimethyl sulfoxide and a bioactive. In one embodiment the base structure of such a device includes a polymer mixed with the bioactive, triacetin and dimethyl sulfoxide. In another embodiment, the bioactive, triacetin and dimethyl sulfoxide are present in a coating on a surface of the device. In this embodiment, the bioactive, triacetin and dimethyl sulfoxide may be contained within a carrier material. The implantable device may also include a porous layer over at least a portion of the coating.

The implantable device may be a stent, a vascular stent, a ureteral stent, a catheter, a balloon, a balloon catheter, a stent graft, a wire guide or a cannula. In one embodiment, the implantable device is a ureteral stent.

The triacetin and the dimethyl sulfoxide
are present at a ratio of between 5:1 w/w and 1:5 w/w pref. at a ratio of
between 2:1 w/w and 1:2 w/w. The triacetin/dimethyl sulfoxide and the bioactive may be present at a ratio of between 5:1 w/w and 1:5 w/w.

In other embodiments, the bioactive is an anesthetic, an analgesic, an antimicrobial agent, an anticancer agent, an antispasmodic agent, an antithrombogenic agent, an anti-inflammatory agent, or an immunosuppressant agent.

Another embodiment provides an implantable device including a polymeric base structure having triacetin, dimethyl sulfoxide and doxazosin mesylate contained within the base structure.

In another aspect, the present invention provides a method for delivering a bioactive to tissue of a patient for the treatment of a disease or condition. The method includes contacting a vessel wall of the patient with one of the compositions or devices described above and maintaining the composition or device in contact with the vessel wall for a time sufficient to deliver the bioactive to the patient. The triacetin and
dimethyl sulfoxide enhances delivery of the bioactive to the tissue. In certain embodiments the vessel is a vascular vessel, an esophageal vessel, an intestinal vessel, a biliary vessel, an urethral vessel or an ureteral vessel. In other embodiments, the bioactive is an anesthetic, an analgesic, an antimicrobial agent, an anticancer agent, an antithrombogenic agent, an anti-spasmodic agent, an anti-inflammatory agent, or an immunosuppressant agent.

### BRIEF DESCRIPTION OF THE DRAWINGS

FIG. 1 depicts a partial cross-sectioned end view of an illustrative embodiment of the implantable medical device.
FIG. 2 depicts a partial cross-sectioned end view of another embodiment of the implantable medical device.

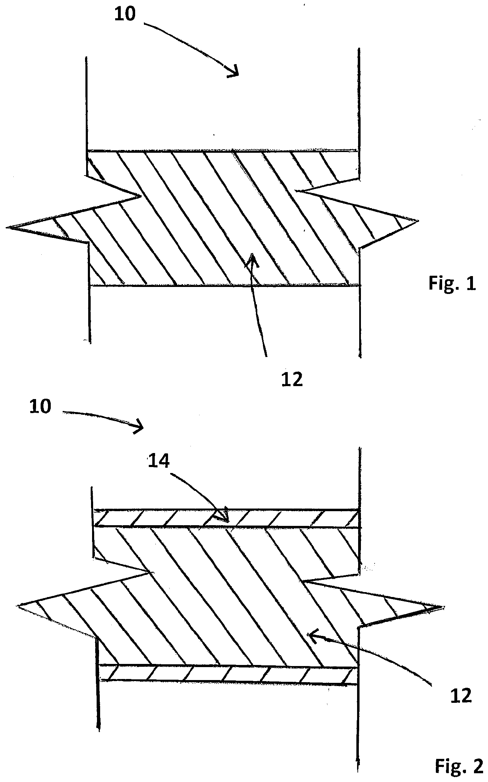

FIG. 2 depicts a partial cross-sectioned end view of yet another embodiment of the implantable medical device.

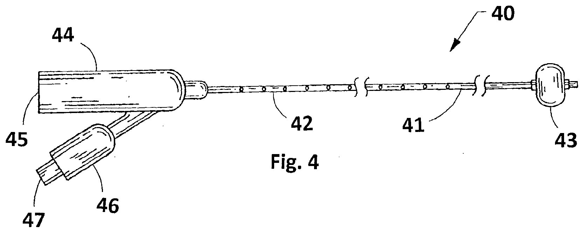

FIG. 4 depicts a plan view of a Foley catheter.

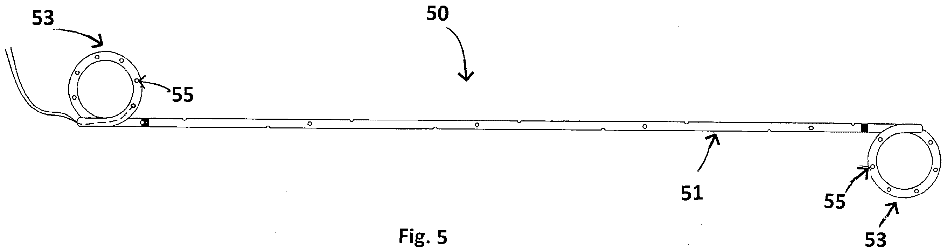

FIG. 5 depicts a plan view of a ureteral stent.

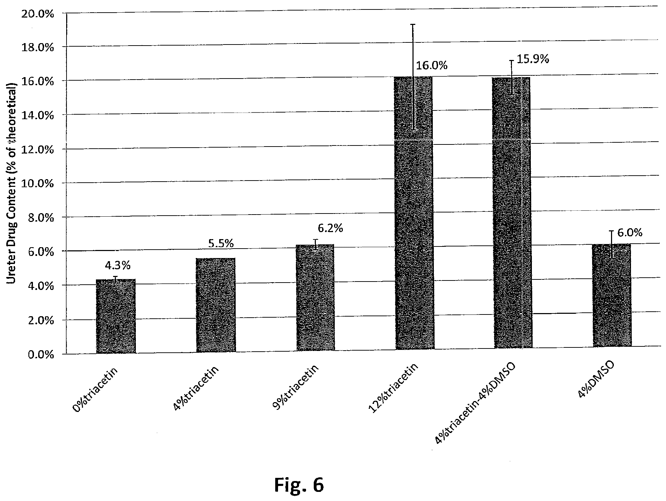

FIG. 6 is a bar chart showing the effect of triacetin and DMSO on the uptake of doxazosin mesylate by porcine ureter implants.

### DETAILED DESCRIPTION OF THE PREFERRED EMBODIMENTS

### Definitions

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example") provided herein, is intended merely to better illuminate the invention and does not pose a limitation on the scope of the invention unless otherwise claimed. No language in the specification should be construed as indicating any non-claimed element as essential to the practice of the invention.

As used herein the terms "comprise(s)," "include(s)," "having," "has," "can," "contain(s)," and variants thereof, are intended to be openended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

As used herein, the term "implantable" refers to an ability of a medical device to be positioned at a location within a body, such as within a body vessel. Furthermore, the terms "implantation" and "implanted" refer to the positioning of a medical device at a location within a body, such as within a body vessel.

The term "bioabsorbable" is used herein to refer to materials that dissipate upon implantation within a body, independent of which mechanisms by which dissipation can occur, such as dissolution, degradation, absorption and excretion. A "non-bioabsorbable" or "biostable" material refers to a material, such as a polymer or copolymer, which remains in the body without substantial bioabsorption.

The term "adapted" for introduction into a human or veterinary patient is used herein to refer to a device having a structure that is shaped and sized for introduction into a human or veterinary patient.

As used herein, the term "body vessel" means any body passage or lumen, including but not limited to blood coronary or peripheral vessels, esophageal, intestinal, biliary, urethral and ureteral passages.

As used herein, the term "bioactive" refers to any agent that produces an intended therapeutic effect on the body to treat or prevent conditions or diseases.

A "therapeutically-effective amount" as used herein is the minimal amount of a bioactive (e.g. doxazosin mesylate) which is necessary to impart therapeutic benefit to a human or veterinary patient. Compositions and Implantable Medical Devices including a Permeation Enhancing Agent

For the purpose of promoting an understanding of the principles of the invention, reference will now be made to embodiments, some of which are illustrated in the drawings, and specific language will be used to describe the same. Any alterations and further modifications in the described embodiments, and any further applications of the principles of the invention as described herein are contemplated as would normally occur to one skilled in the art to which the invention relates.

The present invention relate to compositions
and implantable medical devices including a bioactive and a permeation enhancing agent including triacetin and dimethyl sulfoxide ("DMSO"). The permeation enhancing agent increases the delivery of the bioactive across a vessel wall and into the tissue of the patient.

DMSO acts as a solubilizing agent that dissolves the drug and also enhances the absorption of the drug into the tissues of the patient. DMSO, by itself, is effective at enhancing drug uptake to the tissues. Triacetin is also present. It is believed that triacetin increases the permeability of the membranes of the cells in the vessel wall, resulting in an increase in the amount of drug that the DMSO carries into the tissues. Thus, the triacetin and DMSO work synergistically to enhance tissue drug uptake.

In the discussions that follow, a number of potential features or selections of the bioactive, amounts and ratios of composition components, implantable device structure, or other aspects, are disclosed. It is to be understood that each such disclosed feature or features can be combined with the generalized features discussed, to form a disclosed embodiment of the present invention.

The triacetin and DMSO are present at a ratio of between 5:1 w/w and 1:5 w/w. In yet other embodiments, triacetin and DMSO are present at a ratio of between 2:1 w/w and 1:2 w/w. In other embodiments, triacetin and DMSO are present in at a ratio of approximately 1:1 w/w. In some embodiments, the ratio of permeation enhancing agent(s) to bioactive is between 20:1 w/w and 1:20 w/w or between 10:1 w/w and 1:10 w/w or between 5:1 w/w and 1:5 w/w or between 2:1 w/w and 1:2 w/w or is approximately 1:1 w/w.

The increase in the amount of the bioactive delivered to the tissue of the patient from a composition or device including such permeation enhancing agents may depend upon a number of factors, such as the nature of the vessel in which the composition is placed or device is implanted, as well as the environment within the vessel and the construction of the implantable device.

However, the increase in the amount bioactive delivered through a vessel wall may be characterized in an *ex vivo* assay in which the composition or implantable device is placed in a section of the appropriate vessel and incubated in a buffer solution for a fixed time. In one such assay, a device, for example, is placed in a section of porcine ureter, which is hydrated in a Phosphate Buffered Saline buffer. The ureter is incubated in a closed container for a fixed time, for example 24hrs at 37 deg. C. After the incubation period, the bioactive present in the ureter tissue is extracted using an extraction solution, such as an enzyme solution, organic solvent, organic/aqueous mixture, or acidified mixture. The extraction solution used is dependent on the bioactive being extracted. The amount of bioactive present in the ureter and in the device is determined using, for example, an appropriate HPLC method.

In certain embodiments, the claimed compositions or devices include an amount of triacetin and DMSO sufficient to increase the amount of bioactive delivered, as measured by this assay, by at least 5%, 10%, 15%, 20%, 25%, 30%, 40%, 50%, 70%, 100%, 150% or 200% compared to the amount of bioactive delivered, under the same conditions, from an otherwise identical device that does not include the triacetin and DMSO. In other embodiments, the claimed compositions or devices include an amount of triacetin and DMSO sufficient to increase the amount of bioactive delivered, as measured by this assay, within a period of 1, 5, 15, 30, 60, 120, 300, 500 or 1000 minutes by at least 5%, 10%, 15%, 20%, 25%, 30%, 40%, 50%, 70%, 100%, 150% or 200%. In yet other embodiments, the claimed compositions or devices include an amount of triacetin and DMSO sufficient to increase the amount of bioactive delivered, as measured by this assay, within a period of 1, 5, 15, 30, 60 or 120 days by at least 5%, 10%, 15%, 20%, 25%, 30%, 40%, 50%, 70%, 100%, 150% or 200%.

The medical device may be any of a wide variety of devices having an implantable structure adapted for temporary or permanent implantation in a human or veterinary patient. Medical devices having structures implantable in a body passage will often be used. The body passage may be, for example, a passage of the alimentary system, the urogenital system, the biliary system, or the cardiovascular system.

Medical devices including a structure implantable in the urogenital system are preferred, including, for example, those implantable in the ureter, bladder or urethra. In other embodiments, the device has a structure implantable in a vessel of the cardiovascular system. The passage may be a tubular passage such as an artery or vein, or may be a larger chamber such as a ventricle or atrium of the heart. Implantable medical devices that include structures that span or bridge between bodily passages are also contemplated. The device can be adapted to be entirely or only partially implanted in a bodily passage.

By way of example, the medical device can be or include a catheter, a balloon catheter, a wire guide, a stent, a stent graft, a coil, a needle, a graft, a filter, a balloon, a cutting balloon, a scoring balloon, or any combination of these. The catheter may be, for example, a urethral catheter, a catheter for nephrostomy drainage, a catheter for nasal pancreatic drainage, a catheter for suprapubic drainage, or a nasal biliary drainage catheter. The stent may be, for example, a coronary or peripheral vascular stent, a urethral stent, a prostatic stent, a biliary stent, a pancreatic stent.

Suitable filters include for example vena cava filters such as the Cook CELECT^{®} and Cook Günther TuULIP^{®} and Cook Gianturco-Roehm Bird's NEST^{®} filters available from Cook Medical Incorporated, Bloomington Indiana, USA. Suitable stents include those without a sheath covering, for example the Cook ZILVER^{®} stent available from Cook Medical Incorporated. Suitable stents also include those with a sheath covering. Suitable coils include embolization coils. Suitable wire guides include for instance traditional wire guides as well as wire guides with an attached expandable structure for expansion within a blood vessel lumen, such as a coil, where the expandable structure can optionally carry the coating or coatings as disclosed herein. These or other implants, in certain preferred embodiments, have at least a portion that is configured to expand during deployment so as to contact walls of the passage in which they are implanted to anchor within the passage. In this regard, both self-expanding and force-expandable (e.g. balloon-expandable) stents or other implantable medical devices are contemplated as being within the scope of embodiments of the present invention. As well, it is contemplated that the implantable device may be configured for introduction by a minimally-invasive surgical technique, especially percutaneous introduction, or may be configured for introduction by invasive surgery in which the site of intended implantation in the blood passage is surgically exposed from the exterior of the patient for introduction of the implantable medical device. The implantable medical device may also be percutaneously retrievable, for example a percutaneously retrievable stent, filter or frame (e.g. a spiral frame). These and other variations in the implantable medical device and its associated procedure for introduction will be apparent to those skilled in the pertinent art from the descriptions herein.

The implantable medical device can be made from any suitable material or combination of materials. Illustratively, the implantable medical device can include a metal such as stainless steel, tantalum, titanium, nitinol, cobalt, chromium, nickel, molybdenum, manganese, gold, platinum, inconel, iridium, silver, tungsten, elgiloy, alloys of any of these, or another biocompatible metal; carbon or carbon fiber; a calcium-containing inorganic material such as a ceramic; a material composed of ceramic and metallic components (cermet); or a polymeric material. The material of construction for the implantable medical device structure can be biodegradable or biostable.

The implantable medical device can include biostable polymers, for instance cellulose acetate, cellulose nitrate, silicone, polyethylene terephthalate, polyurethane, polyamide, polyester (e.g. Nylon), polyorthoester, polyanhydride, polyether sulfone, polycarbonate, polypropylene, high molecular weight polyethylene, and polytetrafluoroethylene, or mixtures of these. Biodegradable polymers can also be used, including for instance polylactic acid (PLA), polyglycolic acid (PGA), poly(lactic-co-glycolic acid) (PLGA), polyanhydride, polycaprolactone, polyhydroxybutyrate valerate, hydrogels, natural polymers such as fibrin, collagens, extracellular matrix (ECM) materials, dextrans, polysaccharides and hyaluronic acid, or co-polymers and/or mixtures of these.

The implantable device may include one or more bioabsorbable metals. For example, bioabsorbable metal devices, for example stents, can incorporate bioabsorbable materials such as magnesium, titanium, zirconium, niobium, tantalum, zinc, silicon, lithium, sodium, potassium, calcium, iron, manganese, yttrium, rare earth metals, such as neodymium, or alloys and/or mixtures of two or more of these materials. Some preferred metallic bioabsorbable material alloy compositions include lithium-magnesium, sodium-magnesium, and zinc-titanium. Further details of bioabsorbable metals useful in the manufacture of implantable devices are described in U.S. Patent Publication Number 2010/0262221.

With reference now to Figure 1, this figure shows an implantable medical device 10 including a structure 12 adapted for introduction into a human or veterinary patient. For clarity, only a portion of the structure is shown. By way of example, device 10 is configured as a catheter particularly adapted for insertion into the urinary system of the patient, such as into the urethra.

In certain embodiments, structure 12 includes a polymer incorporating a bioactive and a composition including triacetin and DMSO. Such devices may be prepared, for example, by impregnating structure 12 with the bioactive, triacetin and DMSO. Methods of impregnating polymeric structures are described in U.S. Patent Publication Number 2006/0025726. A mixture of the bioactive, triacetin and DMSO may be impregnated into the polymer structure or, one or more of the three components may be added separately. In some embodiments, one or more of the bioactive, triacetin and DMSO may be impregnated into separate regions of structure 12.

In other embodiments, the bioactive, triacetin and DMSO are incorporated into structure 12 during the formation of the structure, for example, during a molding process. Typically, a mixture of the bioactive, triacetin and DMSO are mixed with a polymeric material, which is then cross linked or dipped cast to form the required structure. The device may also be formed by incorporating certain of the three components into the structure before polymerization and then impregnating the remaining components, as discussed above, after the structure is formed.

Figure 2 illustrates another embodiment showing an implantable medical device 10 including a structure 12 and a coating 14 covering at least part of the structure. A mixture of the bioactive and triacetin and DMSO may be present in coating 14. In certain embodiments, a carrier material, such as a biostable or bioabsorbable polymer, is also present in coating 14. The carrier material may act to control the release of the bioactive when the device is implanted. For example, the carrier material may control the release of the bioactive such that the bioactive is eluted from the device over a longer time that would be the case if the carrier material was not present. Alternatively, the carrier material may act such that the bioactive is eluted more quickly than it would if the carrier material was not present. In other embodiments, no carrier material is present.

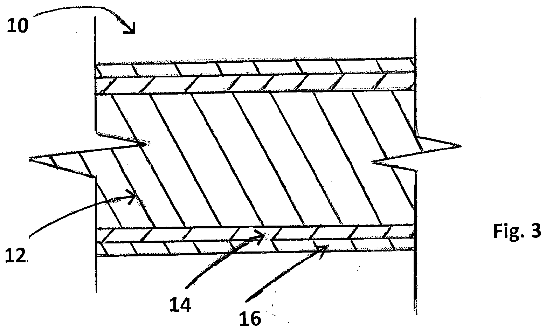

With reference now to Figure 3, this figure shows an implantable medical device 10 including a structure 12, a coating 14 covering at least part of the structure, and an overcoat layer 16 covering at least a portion of coating 14. Layer 16 may be formed from a porous material, for example a polymer, or from a bioabsorbable material, such as a bioabsorbable polymer. Layer 16 may also act to control the release of the bioactive from the device and may also provide protection for the underlying layer(s) during implantation of the device.

The implantable device may include additional coating layers. For example, the device may include multiple coating layers, each containing a bioactive. These layers may be separated from each other by bioactive free layers, for example by porous polymer layers. Alternatively, multiple bioactive containing layers may be deposited directly on top of each other. In some embodiments, one or more of the bioactive(s), triacetin and DMSO may be contained is separate layers. In other embodiments, one or more of the bioactive(s), triacetin and DMSO may be contained within structure 12 while the remaining of these three components may be included in one or more of the coating layers. In yet other embodiments, structure 12 may include apertures, such as holes or wells, containing at least one of the bioactive(s), triacetin and DMSO. Further examples of implantable structures incorporating coatings including elutable bioactives that are applicable to the present embodiments as disclosed in U.S. Patent Number 7,410,665.

Coatings 14 and 16 can be applied to the medical device in any known manner. For example, a coating may be applied by spraying, dipping, pouring, pumping, brushing, wiping, vacuum deposition, vapor deposition, plasma deposition, electrostatic deposition, ultrasonic deposition, epitaxial growth, electrochemical deposition or any other method known to those skilled in the art.

In one embodiment, the coating material is dissolved in a solvent and sprayed onto the medical device under a fume hood using a spray gun, such as the Model Number 200 spray gun manufactured by Badger Air-Brush Company, Franklin Park, IL 60131. Alignment of the spray gun and medical device may be achieved with the use of laser beams, which may be used as a guide when passing the spray gun up and down the medical device being coated.

In another embodiment, the coating material is dissolved in a solvent and then sprayed onto the medical device using an electrostatic spray deposition (ESD) process. The ESD process generally depends on the principle that a charged particle is attracted towards a grounded target.

In yet another embodiment, the medical device is coated using an ultrasonic spray deposition (USD) process. Ultrasonic nozzles employ high frequency sound waves generated by piezoelectric transducers which convert electrical energy into mechanical energy. The transducers receive a high frequency electrical input and convert this into vibratory motion at the same frequency. This motion is amplified to increase the vibration amplitude at an atomizing surface. For example, the medical device can be coated using an ultrasonic spray nozzle, such as those available from Sono-Tek Corporation, Milton, NY 12547.

Figures 4 and 5 illustrate examples of medical devices applicable for use with the present invention. Figure 4 depicts a Foley catheter 40, which includes elongated element 41 for draining urine from a urinary bladder of a patient. The Foley catheter has a constant cross section or diameter for most of the length of elongated element 41, except for a retention balloon 43. Balloon 43 is placed into the patient's bladder and is then inflated using fitting 46 and inflation lumen 47. Urine is drained from the patient through outlet 45 and outlet fitting 44, which may be used to connect to a container, such as a drainage bag. Elongated element 41 may be at least partly formed of a polymer or elastomer.

Figure 5 depicts a view of a ureteral stent. Ureteral stent 50 includes an elongated element 51 connecting curled ends 53. The ureteral stent is implanted such that one of the curled ends 53 is placed in at the top end of the ureter, near the kidney. The second curled end is positioned in the bladder. Urine enters the stent through holes 55 at the curled end positioned near the kidney and travels through a lumen within elongated element 51 to exit the stent through holes 55 at the second curled end and into the bladder.

Such Foley catheters or ureteral stents may contain a region that is impregnated, coated, or otherwise includes a bioactive, such as an antibiotic, another antimicrobial bioactive, or an anesthetic. Triacetin and/or DMSO can be included with the bioactive to enhance the delivery of the bioactive to the tissues of the wall of the urinary vessel.

The claimed implantable devices can include one or more of a wide range bioactives that elute from the device for delivery to the patient. For example, the device may contain at least one of heparin or another thrombin inhibitor, hirudin or another antithrombogenic agent, urokinase or another thrombolytic agent, a fibrinolytic agent, a vasospasm inhibitor, an alpha blocker such as doxazosin mesylate, a calcium channel blocker, nitric or another vasodilator, terazosin or another antihypertensive agents, an anesthetic such as bupivacaine , an analgesic, an anti-spasmodic agent, an antimicrobial agent, an antibiotic, an antiplatelet agent, an antimitotic, a microtubule inhibitor, an actin inhibitor, a remodeling inhibitor, a deoxyribonucleic acid, an antisense polynucleotide, methotrexate or another antiproliferative agent, tamoxifen citrate, a taxane agent such as paclitaxel or a derivative thereof, a mammalian target of rapamycin (mTOR) inhibitor such as sirolimus or a derivative thereof, an anti-cancer agent such as carboplatin, dexamethasone or a dexamethasone derivative, an anti-inflammatory steroid or non-steroidal anti-inflammatory agent, cyclosporin or another immunosuppressive agent, a peptide, a protein, an enzyme, an extracellular matrix component, a cellular component or another biologic agent, captopril, enalapril or another angiotensin converting enzyme (ACE) inhibitor, ascorbic acid, alpha tocopherol, superoxide dismutase, deferoxamine, an iron chelator or antioxidant or mixtures of at least two of these agents.

Another aspect of the invention provides a pharmaceutical composition including triacetin and DMSO in combination with a bioactive and a pharmaceutically-acceptable carrier and the administration of such a pharmaceutical composition to a subject in a manner commensurate with treatment for symptoms associated with a disease or condition. Such pharmaceutical compositions may be formulated, for example, by employing conventional solid or liquid vehicles or diluents, as well as pharmaceutical additives of a type appropriate to the mode of desired administration (for example, excipients, binders, preservatives, stabilizers, flavors) according to techniques such as those well known in the art of pharmaceutical formulation.

The pharmaceutical compositions can be administered by any suitable means. For example, they can be administered by subcutaneous, intravenous, intramuscular, or intracisternal injection or infusion techniques (e.g., as sterile injectable aqueous or non-aqueous solutions or suspensions; as sterile injectable aqueous or non-aqueous solutions delivered by an infusion catheter); nasally, such as by inhalation spray; or topically, such as in the form of a cream or ointment; orally and in dosage unit formulations containing non-toxic, pharmaceutically-acceptable vehicles or diluents. Such pharmaceutical compositions can, for example, be administered in a form suitable for immediate release or extended release. Immediate release or extended release may be achieved by the use of suitable pharmaceutical compositions, or, particularly in the case of extended release, by the use of devices such as subcutaneous implants or osmotic pumps. The pharmaceutical compositions may also be administered in the form of liposomes.

Oral pharmaceutical compositions generally include an inert diluent or an edible carrier. They can be enclosed in gelatin capsules or compressed into tablets. For the purpose of oral therapeutic administration, the pharmaceutical composition may include excipients and be in the form of tablets, troches, or capsules. Oral compositions can also be prepared using a fluid carrier for use as a mouthwash, wherein the compound in the fluid carrier is applied orally. Pharmaceutically compatible binding agents, and/or adjuvant materials can be included. Tablets, pills, capsules, troches and the like can contain, for example, the following ingredients, or compounds of a similar nature: a binder such as microcrystalline cellulose, gum tragacanth or gelatin; an excipient such as starch or lactose, a disintegrating agent such as alginic acid or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

### Methods of Delivery and Treatment

Another aspect of the present invention provides methods of delivering a therapeutically effective amount of a bioactive to the tissue of a patient for the purposes to treating a disease or condition. The bioactive may be delivered as a pharmaceutical composition, such as those disclosed above, or in combination with the implantable devices disclosed above. The pharmaceutical composition may be delivered directly to a vessel of a patient. In one embodiment, the pharmaceutical composition is delivered to the urinary system, for example to the bladder, of a patient by, for example, infusion through a catheter.

In one embodiment, a fluid including a chemotherapeutic drug is infused into the urethra or other body passage of a patient and maintained in contact with the passage wall for a time period sufficient to deliver a therapeutically effective amount of the chemotherapeutic drug to the surrounding tissue. In one embodiment, the permeation enhancing agent, including triacetin and DMSO, is present, along with the chemotherapeutic drug, in the infusion fluid and provides for a more rapid delivery of the chemotherapeutic drug to the tissue, as compared to the time required to deliver an equivalent amount of drug in the absence of the permeation enhancing agent. For example, the time required to deliver the required amount of the drug may be decreased by 10, 20, 30, 50 or 70 percentage.

In certain other embodiments, the method includes the steps of inserting an implantable medical device having any of the novel configurations described above into the patient and maintaining the device in position for a time sufficient to deliver a bioactive to the tissue of the patient. For example, the bioactive may be delivered locally to a passage of the alimentary system, the urogenital system, the biliary system, or the cardiovascular system. The permeation enhancing agent, including triacetin and DMSO, is present on or within the device and provides for the delivery of an increased amount of the bioactive to the tissue of the patient, compared to the amount of bioactive delivered from a similar device not including the permeation enhancing agent.

In certain embodiments, the device or pharmaceutical composition includes an amount of triacetin and DMSO sufficient to increase the amount of bioactive delivered to the tissue of the patient by at least 5%, 10%, 15%, 20%, 25%, 30%, 40%, 50%, 70%, 100%, 150% or 200%. In other embodiments, the device or pharmaceutical composition includes an amount of triacetin and DMSO sufficient to increase the amount of bioactive delivered to the tissue of the patient within a period of 1, 5, 15, 30, 60, 120, 300, 500 or 1000 minutes by at least 5%, 10%, 15%, 20%, 25%, 30%, 40%, 50%, 70%, 100%, 150% or 200%. In yet other embodiments, the claimed device or composition includes an amount of triacetin and DMSO sufficient to increase the amount of bioactive delivered to the tissue of the patient within a period of 1, 5, 15, 30, 60 or 120 days by at least 5%, 10%, 15%, 20%, 25%, 30%, 40%, 50%, 70%, 100%, 150% or 200%.

For example, when the implantable medical device is a vascular stent, the method of treatment involves implanting the stent into the coronary or peripheral vascular system of a patient for a time sufficient to deliver the bioactive(s) from the stent. In certain embodiments, the bioactive is a taxane, taxane analogue or derivative thereof, for example paclitaxel. In one embodiment, the device is implanted to treat peripheral vascular disease, for example by implanting the device in a peripheral artery.

In another embodiment, the device is a urethral or ureteral catheter or stent, for example, a Foley catheter introduced into the patient's bladder via the urethra for the drainage of urine. In such an embodiment, an antimicrobial agent, such as chlorhexidine, may be included in or on the device to prevent or treat any infection associated with the implantation of the device. In yet another embodiment, an anesthetic agent, such as bupivacaine, may be incorporated into the device to reduce or eliminate pain associated with the placement of the device. In another embodiment, an Alpha blocker, such as doxazosin mesylate, may be incorporated into the device for the treatment of benign prostatic hyperplasia or tissue spasming.

### Examples

### Example 1 - Preparation of tubing containing doxazosin mesylate, triacetin and DMSO

Polyurethane pellets are added to a 90:10 (v:v) mixture of methanol:methylene chloride. The solution is stirred until the polyurethane pellets are dissolved. Triacetin, if required, is then added to this solution and the resulting mixture stirred until the triacetin is dissolved. A mixture of doxazosin mesylate and DMSO is sonicated until the doxazosin mesylate is dissolved. The doxazosin mesylate/DMSO mixture is combined with the polyurethane solution and the two solutions mixed. If no DMSO is required, the doxazosin mesylate is dissolved in the polyurethane solution.

Polyurethane polymer tubing (6Fr.) is coated with the resulting solution by dipping into the coating solution three times with at least 2 minutes of air drying between dips. After the final dip, the device is dried for at least 12 hours. The coating solution contains approximately 4 milligrams/ml doxazosin mesylate and either (i) no triacetin or DMSO, (ii) 4% triacetin, (iii) 9% triacetin, (iv) 12% triacetin, (v) 4% triacetin and 4% DMSO, or (vi) 4% DMSO.

### Example 2 - Ex-vivo delivery of doxazosin mesylate to ureter tissue

A 7cm section of each of the samples of tubing prepared as described in Example 1 is inserted into 9cm of porcine ureter and incubated for approximately 18 hours at 37°C in a screw capped glass vial hydrated with 0.25ml of PBS (pH=7.4). The glass vials are incubated horizontally, so that the ureter sits directly in the PBS. The amount of doxazosin mesylate present in the tubing and in the ureter is determined as described in example 3.

### Example 3 - Determination of the enhancement of delivery of doxazosin mesylate to ureter tissue by triacetin and DMSO

Each of the samples from Example 2 is tested to determine the amount of doxazosin mesylate delivered to the ureter tissue. Doxazosin mesylate is extracted from each tube and ureter and the percentage that is delivered to each ureter is determined as follows. The tube and ureter are incubated separately in an organic solvent to extract the doxazosin mesylate present in each material. The amount of doxazosin mesylate in each of the ureter and the tubing is measured using HPLC.

Figure 6 shows the percentage of doxazosin mesylate present in the ureter for each of the conditions described above. Both triacetin and DMSO increase the amount of doxazosin mesylate delivered. The presence of both compounds results in a synergistic delivery to the ureter. The largest increase in amount delivered is obtained using either 12% triacetin or a mixture of 4% triacetin/4% DMSO.

Although the invention has been described and illustrated with reference to specific illustrative embodiments thereof, it is not intended that the invention be limited to those illustrative embodiments.

## Claims

1. An implantable device comprising:
a base structure,
a composition comprising triacetin and dimethyl sulfoxide, and
a bioactive,
wherein the composition and the bioactive are in contact with or contained within the base structure, and wherein the triacetin and the dimethyl sulfoxide are present at a ratio of between 5:1 w/w and 1:5 w/w.

2. The implantable device of claim 1, wherein the base structure comprises a polymer and wherein the bioactive and the composition form a mixture with the polymer.

3. The implantable device of claim 1, wherein the base structure comprises a surface and wherein the bioactive and the composition are present on the surface.

4. The implantable device of claim 3, wherein the bioactive and the composition are contained within a carrier material.

5. The implantable device of claim 3, further comprising a porous layer over at least a portion of the bioactive and the composition.

6. The implantable device of claim 1, wherein at least one of the composition and the bioactive form a coating on at least one surface of the base structure and wherein the coating is free of a carrier material.

7. The implantable device of claim 1, wherein one of the composition and the bioactive is incorporated within the base structure and the other of the composition and the bioactive forms a coating on a surface of the base structure.

8. The implantable device of any one of the preceding claims, wherein the implantable device is selected from the group consisting of a stent, a vascular stent, a ureteral stent, a catheter, a balloon, a balloon catheter, a stent graft, a wire guide, and a cannula.

9. The implantable device of any one of the preceding claims, wherein the triacetin and the dimethyl sulfoxide are present at a ratio of between 2:1 w/w and 1:2 w/w.

10. The implantable device of any one of the preceding claims, wherein the base structure comprises a metal.

11. The implantable device of any one of the preceding claims, wherein the bioactive is selected from the group consisting of an anesthetic, an analgesic, an antimicrobial agent, an anticancer agent, an antithrombogenic agent, an anti-apasmodic agent, an anti-inflammatory agent, and an immunosuppressant agent.

12. The implantable device of any one of the preceding claims, wherein the composition and the bioactive are present at a ratio of between 5:1 w/w and 1:5 w/w.

13. The implantable device of claim 1 wherein the base structure comprises:
a polymeric base structure,
wherein the composition comprises triacetin, dimethyl sulfoxide and doxazosin mesylate, and
wherein the composition is contained within the polymeric base structure.

14. A composition comprising triacetin, dimethyl sulfoxide, a bioactive and a pharmaceutically acceptable carrier, wherein the triacetin and the dimethyl sulfoxide are present at a ratio of between 5:1 w/w and 1:5 w/w.

15. The use of the composition of claim 14 in the manufacture of a medical device.

## Patentansprüche

1. Implantierbare Vorrichtung, die umfasst:
eine Grundstruktur,
eine Zusammensetzung, die Triacetin und Dimethylsulfoxid umfasst, und
eine bioaktive Verbindung,
wobei die Zusammensetzung und die bioaktive Verbindung in Kontakt mit der Grundstruktur sind oder darin enthalten sind, und wobei das Triacetin und das Dimethylsulfoxid in einem Verhältnis von zwischen 5:1 (w/w) und 1:5 (w/w) vorliegen.

2. Implantierbare Vorrichtung nach Anspruch 1, wobei die Grundstruktur ein Polymer umfasst und wobei die bioaktive Verbindung und die Zusammensetzung ein Gemisch mit dem Polymer bilden.

3. Implantierbare Vorrichtung nach Anspruch 1, wobei die Grundstruktur eine Oberfläche umfasst und wobei die bioaktive Verbindung und die Zusammensetzung auf der Oberfläche vorliegen.

4. Implantierbare Vorrichtung nach Anspruch 3, wobei die bioaktive Verbindung und die Zusammensetzung in einem Trägermaterial enthalten sind.

5. Implantierbare Vorrichtung nach Anspruch 3, die weiter eine poröse Schicht über mindestens einem Teil der bioaktiven Verbindung und der Zusammensetzung umfasst.

6. Implantierbare Vorrichtung nach Anspruch 1, wobei mindestens eins von der Zusammensetzung und der bioaktiven Verbindung eine Beschichtung auf mindestens einer Oberfläche der Grundstruktur bildet und wobei die Beschichtung frei von Trägermaterial ist.

7. Implantierbare Vorrichtung nach Anspruch 1, wobei eins von der Zusammensetzung und der bioaktiven Verbindung in die Grundstruktur eingearbeitet ist und das andere von der Zusammensetzung und der bioaktiven Verbindung eine Beschichtung auf einer Oberfläche der Grundstruktur bildet.

8. Implantierbare Vorrichtung nach einem der vorhergehenden Ansprüche, wobei die implantierbare Vorrichtung aus der Gruppe ausgewählt ist, die aus einem Stent, einem Gefäß-Stent, einem Ureter-Stent, einem Katheter, einem Ballon, einem Ballonkatheter, einem Stentgraft, einem Führungsdraht, und einer Kanüle besteht.

9. Implantierbare Vorrichtung nach einem der vorhergehenden Ansprüche, wobei das Triacetin und das Dimethylsulfoxid in einem Verhältnis von zwischen 2:1 (w/w) und 1:2 (w/w) vorliegen.

10. Implantierbare Vorrichtung nach einem der vorhergehenden Ansprüche, wobei die Grundstruktur ein Metall umfasst.

11. Implantierbare Vorrichtung nach einem der vorhergehenden Ansprüche, wobei die bioaktive Verbindung aus der Gruppe ausgewählt ist, die aus einem Anästhetikum, einem Analgetikum, einem Antimikrobiotikum, einem Antikrebsmittel, einem Antithrombotikum, einem Antispasmodikum, einem Antiphlogistikum und einem Immunsuppressivum besteht.

12. Implantierbare Vorrichtung nach einem der vorhergehenden Ansprüche, wobei die Zusammensetzung und die bioaktive Verbindung in einem Verhältnis von zwischen 5:1 (w/w) und 1:5 (w/w) vorliegen.

13. Implantierbare Vorrichtung nach Anspruch 1, wobei die Grundstruktur umfasst:
eine polymere Grundstruktur,
wobei die Zusammensetzung Triacetin, Dimethylsulfoxid und Doxazosinmesylat umfasst, und
wobei die Zusammensetzung in der polymeren Grundstruktur enthalten ist.

14. Zusammensetzung, die Triacetin, Dimethylsulfoxid, eine bioaktive Verbindung und einen pharmazeutisch unbedenklichen Träger umfasst, wobei das Triacetin und das Dimethylsulfoxid in einem Verhältnis von zwischen 5:1 (w/w) und 1:5 (w/w) vorliegen.

15. Verwendung der Zusammensetzung nach Anspruch 14 bei der Herstellung eines Medizinproduktes.

## Revendications

1. Dispositif implantable comprenant :
une structure de base,
une composition comprenant de la triacétine et du diméthylsulfoxyde, et
un bioactif
dans lequel la composition et le bioactif sont en contact avec ou contenus à l'intérieur de la structure de base, et dans lequel la triacétine et le diméthylsulfoxyde sont présents à un rapport entre 5 : 1 p/p et 1 : 5 p/p.

2. Dispositif implantable selon la revendication 1, dans lequel la structure de base comprend un polymère et dans lequel le bioactif et la composition forment un mélange avec le polymère.

3. Dispositif implantable selon la revendication 1, dans lequel la structure de base comprend une surface et dans lequel le bioactif et la composition sont présents sur la surface.

4. Dispositif implantable selon la revendication 3, dans lequel le bioactif et la composition sont contenus à l'intérieur d'un matériau support.

5. Dispositif implantable selon la revendication 3, comprenant en outre une couche poreuse sur au moins une portion du bioactif et de la composition.

6. Dispositif implantable selon la revendication 1, dans lequel au moins l'un de la composition et du bioactif forme un revêtement sur au moins une surface de la structure de base et dans lequel le revêtement est dépourvu de matériau support.

7. Dispositif implantable selon la revendication 1, dans lequel l'un de la composition et du bioactif est incorporé à l'intérieur de la structure de base et l'autre de la composition et du bioactif forme un revêtement sur une surface de la structure de base.

8. Dispositif implantable selon l'une quelconque des revendications précédentes, dans lequel le dispositif implantable est sélectionné dans le groupe consistant en une endoprothèse, une endoprothèse vasculaire, une endoprothèse urétérale, un cathéter, un ballonnet, un cathéter à ballonnet, une greffe d'endoprothèse, un fil-guide et une canule.

9. Dispositif implantable selon l'une quelconque des revendications précédentes, dans lequel la triacétine et le diméthylsulfoxyde sont présents à un rapport entre 2 : 1 p/p et 1 : 2 p/p.

10. Dispositif implantable selon l'une quelconque des revendications précédentes, dans lequel la structure de base comprend un métal.

11. Dispositif implantable selon l'une quelconque des revendications précédentes, dans lequel le bioactif est sélectionné dans le groupe consistant en un anesthésique, un analgésique, un agent antimicrobien, un agent anticancéreux, un agent antithrombogénique, un agent antispasmodique, un agent anti-inflammatoire et un agent immunosuppresseur.

12. Dispositif implantable selon l'une quelconque des revendications précédentes, dans lequel la composition et le bioactif sont présents à un rapport entre 5 : 1 p/p et 1 : 5 p/p.

13. Dispositif implantable selon la revendication 1, dans lequel la structure de base comprend :
une structure de base polymérique,
dans lequel la composition comprend de la triacétine, du diméthylsulfoxyde et du mésylate de doxazosine, et dans lequel la composition est contenue à l'intérieur de la structure de base polymérique.

14. Composition comprenant de la triacétine, du diméthylsulfoxyde, un bioactif et un support pharmaceutiquement acceptable, dans laquelle la triacétine et le diméthylsulfoxyde sont présents à un rapport entre 5 : 1 p/p et 1 : 5 p/p.

15. Utilisation de la composition selon la revendication 14, dans la fabrication d'un dispositif médical.
